Europäisches Patentamt

European Patent Office

Office européen des brevets

(19)

(11)  **EP 0 704 422 A1**

(12)  **EUROPÄISCHE PATENTANMELDUNG**

(43) Veröffentlichungstag:
03.04.1996  Patentblatt 1996/14

(51) Int. Cl.$^6$: **C07C 49/175**,  C07C 45/64,
C07C 45/84

(21) Anmeldenummer: 95115437.6

(22) Anmeldetag: 29.09.1995

(84) Benannte Vertragsstaaten:
BE DE ES FR GB IT NL

(30) Priorität: 30.09.1994 DE 4435176

(71) Anmelder: BASF AKTIENGESELLSCHAFT
D-67056 Ludwigshafen (DE)

(72) Erfinder:
 • Gröning, Carsten, Dr.
  D-68259 Mannheim (DE)
 • Ebel, Klaus, Dr.
  D-68623 Lampertheim (DE)
 • Kaibel, Gerd, Dr.
  D-68623 Lampertheim (DE)
 • Therre, Jörg, Dr.
  D-67550 Worms (DE)

 • Koopmann, Jürgen, Dr.
  D-67434 Neustadt (DE)
 • Menig, Helmuth, Dr.
  D-67159 Friedelsheim (DE)
 • Fritz, Gerard, Dr.
  D-67125 Dannstadt-Schauernheim (DE)
 • Dietz, Rainer
  D-67434 Neustadt (DE)

(74) Vertreter: Geissler, Bernhard, Dr. jur., Dipl.-Phys.
et al
Patent- und Rechtsanwälte
Bardehle . Pagenberg . Dost . Altenburg .
Frohwitter . Geissler & Partner
Postfach 86 06 20
D-81633 München (DE)

(54)  **Verfahren zur Herstellung von Methylglyoxaldimethylacetal**

(57)  Ein Verfahren zur Herstellung von Methylglyoxaldimethylacetal aus Methylglyoxal und Methanol in
Gegenwart eines Katalysators ist dadurch gekennzeichnet, daß das gebildete Methylglyoxaldimethylacetal mit
Hilfe von azeotroper destillierender Erwärmung in
Gegenwart von Wasser gewonnen wird.

## Beschreibung

Diese Erfindung betrifft ein Verfahren zur Herstellung von Methylglyoxaldimethylacetal (1,1-Dimethoxy-2-propanon) durch Umsetzung von Methylglyoxal (2-Oxopropanal) mit Methanol in Gegenwart eines sauren Katalysators, insbesondere eines sauren lonenaustauschers.

Bei den bisher beschriebenen Herstellungen von Methylglyoxaldimethylacetal (MGDA) aus Methylglyoxal (MG) und Methanol wurde häufig versucht, das Wasser, das sowohl aus der Ausgangs-Methylglyoxallösung als auch aus der Umsetzung selbst stammen kann, mit Schleppmitteln während der Reaktion aus dem Ansatz zu entfernen (z. B. Braude et al., J. Chem. Soc. 3321 (1955); Hoechst AG, DE 29 47 383)). Dies erfordert den Einsatz leichtflüchtiger; meist giftiger Hilfsstoffe wie beispielsweise Benzol, die das Produkt verunreinigen können. Die beschriebenen Ausbeuten liegen zudem nur bei maximal 67%. Als Abfallprodukt wird das 1,2,2,2-Tetramethoxypropan beschrieben.

Weiter wird in einer japanischen Patentanmeldung (JP 59199651A) die Acetalisierung von Methylglyoxal mit Methanol an lonenaustauschern in Suspensionen beschrieben, wobei die Aufarbeitung über Extraktion mit Methylenchlorid als Lösungsmittel erfolgt. Da Methylglyoxaldimethylacetal zum großen Teil für die Herstellung von pharmazeutischen Produkten verwendet wird, erscheint der Einsatz dieses giftigen Hilfsstoffes bedenklich. Über den Verbleib des lonenaustauschers und der Destillationsrückstände wird keine Aussage gemacht. Zudem zeigen eigene Experimente (siehe Vergleichsbeispiel), daß die in JP 59199651A angegebenen Ausbeuten mit dem in der Technik vorliegenden Methylglyoxal (ca. 40%ige wäßrige Lösung) nicht nachvollzogen werden können. Die in dieser Druckschrift in den Beispielen verwendeten Methanol-Überschüsse von 1:23 und die langen Reaktionszeiten von 16 Stunden sind außerdem für eine technische Methylglyoxaldimethylacetal-Synthese von Nachteil.

Aufgabe der vorliegenden Erfindung war es deshalb, den zuvor genannten Nachteilen bei der Herstellung von Methylglyoxaldimethylacetal aus Methylglyoxal und Methanol abzuhelfen.

Demgemäß wurde ein neues und verbessertes Verfahren zur Herstellung von Methylglyoxaldimethylacetal nach Anspruch 1 entwickelt, bei dem nach der Umsetzung von Methylglyoxal und Methanol in Gegenwart eines Katalysators das Produkt Methylglyoxaldimethylacetal durch azeotrope Destillation mit Wasser gewonnen wird.

Das Verfahren kann kontinuierlich oder bevorzugt diskontinuierlich durchgeführt werden. Bevorzugt wird dabei Methylglyoxal, das mehr als 40 Gew.-% Wasser enthält, zur Durchführung des weiteren Verfahrens durch Abdestiltieren von Wasser unter reduziertem Druck auf einen Wassergehalt von weniger als 40 Gew.-% angereichert. Weitere bevorzugte Ausführungsformen sind in den Unteransprüchen angegeben.

Das in der vorliegenden Erfindung verwendete Methylglyoxal ist in der Regel technisches Methylglyoxal, das ca. 40 Gew.-% Wasser enthält. Bevorzugt wird diese wäßrige Methylglyoxallösung durch Aodestillieren von Wasser bei 20 - 1013 mbar, besonders bevorzugt bei 50 - 150 mbar, auf Wassergehalte von weniger als 40 Gew.-%, besonders bevorzugt weniger als 20 Gew.-%, in bezug auf Methylglyoxal konzentriert, wobei bei Entwässerung auf Wassergehalte von weniger als 5% schwerflüchtige Lösungsmittel wie z. B. höhersiedende Alkohole (z. B. Decanol oder Diethylenglykol) oder höhersiedende Ether (z. B. Diethylenglykolbutylether) oder ähnlich hochsiedende Lösungsmittel zur Verbesserung der Rührbarkeit zugesetzt werden können.

Die eigentliche Umsetzung von Methylglyoxal mit Methanol findet bevorzugt bei einem molaren Verhältnis von Methylglyoxal:Methanol von 1:2 bis 1:50, bevorzugt 1:8 bis 1:15, statt.

Die Katalyse der Umsetzung z. B. erfolgt auf herkömmliche Weise, beispielsweise bei stark sauren pH-Werten. Bevorzugt werden als derartige saure Katalysatoren saure lonenaustauscher in Form von Festbetten oder als Aufschlämmung eingesetzt, wie sie im Handel erhältlich sind (beispielsweise BayKat 2611, Amberlyst 15 usw.). Die Temperaturen bei der Umsetzung an den Katalysatoren beträgt vorzugsweise 20 bis 120°C, besonders bevorzugt 50 bis 90°C. Die Reaktion muß nicht notwendigerweise zu einem 100%igen Umsatz führen, und dementsprechend kann die Zeitdauer der Umsetzung an den lonenaustauschern variieren.

Eine bevorzugte Reaktionszeit beträgt 4 bis 7 Stunden, insbesondere 5 bis 6 Stunden, bevorzugte Reaktionstemperaturen sind 50 bis 100°C, insbesondere 60 bis 80°C.

Zur Gewinnung des Produkts wird der Reaktionsaustrag mit Wasser versetzt; anschließend wird eine fraktionierte Destillation durchgeführt, wobei überraschenderweise Methylglyoxaldimethylacetal durch das zugesetzte Wasser nicht hydrolysiert wird, obwohl der pH-Wert des Reaktoraustrags sauer ist (pH beispielsweise 2,0); weiter wird ebenfalls entstandenes 1,1,2,2-Tetramethoxypropan, das ein unerwünschtes Nebenprodukt ist, zum gewünschten Produkt Methylglyoxaldimethylacetal hydrolysiert. Vorteilhafterweise wird bei der fraktionierten Destillation des Reaktionsproduktes nach ABtrennung der Leichtsieder und des überschüssigen Methanols zunächst ein einphasiges Gemisch aus MGDA-H$_2$O-Methanol solange abgenommen, bis MeOH weitestgehend aus dem Reaktionsaustrag abgetrennt ist. Das anschließend übergehende Azeotrop von Methylglyoxaldimethylacetal und Wasser (das noch etwas Methanol enthalten kann) zerfällt am Kopf in zwei Phasen, wodurch rohes Methylglyoxaldimethylacetal auf einfache Weise gewonnen werden kann.

Wie bereits erwähnt kann die Reaktion kontinuierlich oder bevorzugt diskontinuierlich durchgeführt werden. Bei der Durchführung werden sämtliche am Verfahren beteiligten Stoffe, die noch Methylglyoxaldimethylacetal, Methylglyoxal oder in Methylglyoxal überführbare Produkte enthalten, immer wieder in das Verfahren zurückgeführt, bis nach mehreren Verfahrensschritten, beispielsweise 12 Verfahrensschritten, schließlich eine Ausbeute von typischerweise über 80% an Roh-Methylglyoxaldimethylacetal erreicht wird.

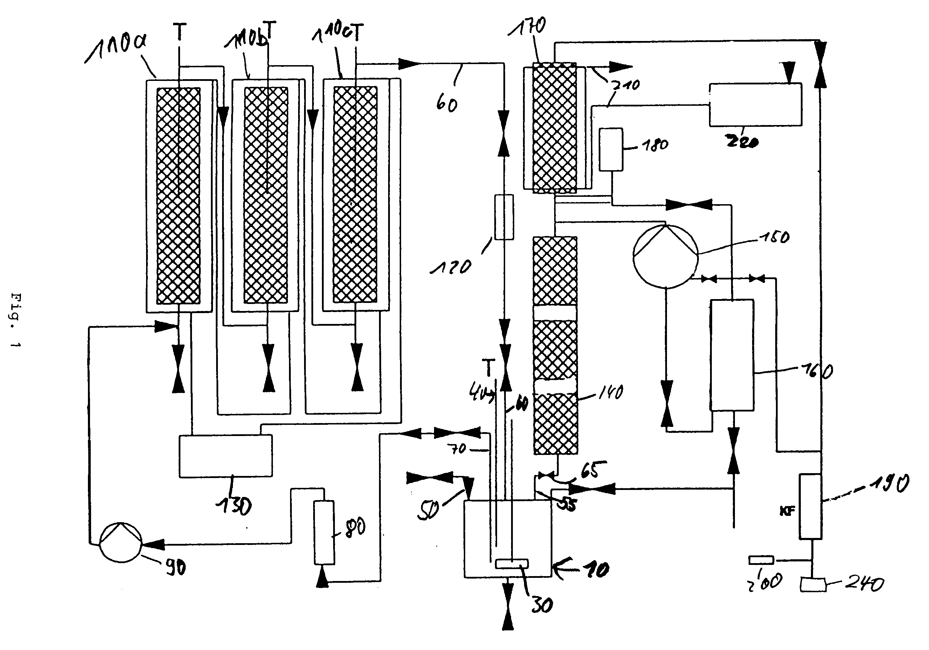

Figur 1 zeigt eine geeignete Apparatur für eine diskontinuierliche Durchführung des erfindungsgemäßen Verfahrens. Ein Rührkessel 10 ist mit einem Rührer 30, einem Temperaturfühler 40, einer Entnahmeleitung 70, einer Einfüllvorrichtung 50 und einer Zuführleitung 60 versehen. Die Entnahmeleitung 70 führt über einen Durchflußmesser 80, eine Pumpe 90 zu den Reaktoren 110a, 110b und 110c, die saure Ionenaustauscherfestbetten sind, welche durch Temperierung mittels des Thermostaten 130 auf einer gewünschten Temperatur gehalten werden. Nach der gewünschten Reaktionszeit wird der sich im Rührkessel befindende Austrag über die Kolonne 140 destilliert. Die Destillationsvorrichtung ist von herkömmlicher Art mit einem Rückflußkühler 170, einer Wasserkühlung 210, deren Temperatur mittels des Thermostaten 220 geregelt wird, einer Pumpe 150, einer Vorlage 160, einem Rückflußteiler 180, einer Kühlfalle 190, einem Druckregler 200 und einer Vakuumpumpe 240. Eine mit Ventil 65 versehene Rückführleitung 55 ermöglicht die Rückführung des Kolonnensumpfes zum Rührkessel.

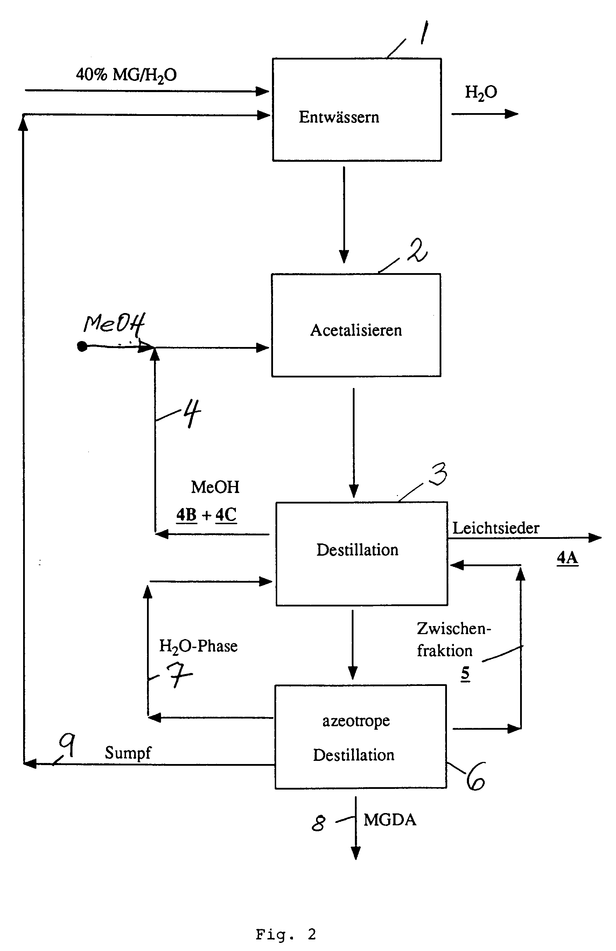

Figur 2 stellt ein Grundfließbild für eine mögliche diskontinuierliche Durchführung des erfindungsgemäßen Verfahrens dar. In Schritt 1 wird industrielles Methylglyoxal (MG) durch Abdestillieren von Wasser konzentriert. Vor Schritt 2 werden frisches Methanol und gegebenenfalls recycliertes Methanol zugesetzt, in Schritt 2 findet die Acetalisierung statt. Der Reaktoraustrag wird mit $H_2O$ versetzt und dann fraktioniert destilliert. Im Destillationsschritt 3 werden leichtsiedende Fraktionen 4A, die ausgeschleust werden, sowie hauptsächlich Methanol enthaltende Fraktionen 4B, die in den nächsten Ansatz recycliert werden, abdestilliert. Anschließend wird eine Zwischenfraktion 5, bestehend aus einem einphasigen Gemisch aus MeOH, MGDA und $H_2O$, abdestilliert. Diese Fraktion wird nach der Acetalisierung in den nächsten Reaktionsschritt zurückgeführt. Danach wird das Methylglyoxal in Stufe 6 azeotrop ausgekreist, wobei das Azeotrop am Destillationskopf in zwei Phasen, nämlich eine wäßrige Phase 7, die während der Destillation wieder in den Sumpf der Destillation 3 zurückgeführt wird, sowie eine Roh-Methylglyoxaldimethylacetal-Phase zerfällt, die als Produkt 8 (MGDA) abgenommen wird. Der Sumpf 9 der Destillation wird nach Beendigung eines derartigen Verfahrensschrittes mit diskontinuierlicher Verfahrensweise wieder in das gegebenenfalls zu entwässernde Ausgangsmaterial (Entwässerung (1) des nächsten Schrittes, falls erforderlich, recycliert.

Beispielhafte Bedingungen zur Verfahrensdurchführung sind ein Druck von ca. 100 mbar, bei dem beispielsweise aus einer wäßrigen technischen Methylglyoxallösung (circa 40 Gew.-% Wasser) das enthaltende Wasser zum größten Teil abdestilliert wird. Nach Versetzen mit Methanol wird dann bevorzugt bei 65-75°C beispielsweise 5 bis 7 Stunden lang über handelsübliche saure Ionenaustauscher (z.B. BayKat. 2611, Amberlyst 15) gepumpt. Bei Temperaturen über dem Siedepunkt des Methanols wird unter dem Eigendruck des Systems gearbeitet. Zunächst werden Methanol und andere niedrigsiedende Fraktionen abgetrennt, bis im Kolonnenkopf Phasenzerfall eintritt, so daß das Methylglyoxaldimethylacetal als Hetero-Azeotrop ausgekreist werden kann. Der verbleibende Sumpf enthält praktisch kein Methylglyoxaldimethylacetal (weniger als 0,3 Gew.-%); er wird in den nächsten Verfahrensschritt zurückgeführt. Dadurch kann überraschenderweise die Ausbeute an Methylglyoxaldimethylacetal erhöht werden, da die im Sumpf enthaltenen Substanzen mit Methanol ebenfalls Methylglyoxaldimethylacetal bilden, wie in Verfahrensstufen, bei denen kein frisches Methylglyoxal zugesetzt wurde, gezeigt wurde.

Die folgenden Beispiele erläutern die Erfindung.

Beispiel 1A

In einem 6 l-Rührkessel werden 438 g Diethylenglykol (4,125 Mol) vorgelegt und mit 986,5 g 40,1%igem Methylglyoxal (5,5 mol) versetzt.

Bei 100 mbar wird die Phase bis zu einer Sumpftemperatur von 80°C abdestilliert. Danach liegt der Wassergehalt in Sumpf bei 3 bis 5 Gew.-%. Man versetzt mit 1760 g (55 Mol) Methanol nach Abkühlen, dann wird die Reaktionsmischung unter dem Eigendruck des Systems 5 Stunden bei 70°C über das Ionenaustauscherbett in den 3 Reaktoren 110a, 110b, 110c gepumpt. Der Eigendruck, der in der gesamten Apparatur herrscht (Ventil zur Kolonne ist geschlossen), beträgt circa 0,8 bar.

Nach Schließen der Ventile zum Reaktor wird der Reaktionsaustrag im Rührkessel mit 500 ml Wasser versetzt, dann wird destilliert. Bis zum Erreichen einer Kopftemperatur von 60°C wird die Leichtsiederfraktion 4A der Figur 3 abdestilliert.

Danach wird bei variablem Rücklauf Methanol (Fraktion 4B) abdestilliert, bis im Sumpf 85°C erreicht sind, wobei die Kopftemperatur bei 65°C festgehalten wird. Das Rücklaufverhältnis wird variabel bis zu einem Verhältnis von 5:1 gehalten.

Dann werden 500 mbar Vakuum angelegt und bei einem variablen Rücklaufverhältnis bis 10:1 wird weiter Methanol abdestilliert (Fraktion 4C; Siedegrenze am Kopf = 49°C). Die Fraktionen 4B und 4C werden vereinigt und als Rückmethanol ( 4 = (4B + 4C) ) vor der Umsetzung am Katalysator in den nächsten Ansatz zurückgeführt. Anschließend wird die Kopftemperatur auf 74,5°C begrenzt und die Zwischenfraktion 5, die aus einem einphasigen Methylglyoxaldimethylacetal-Wasser-Methanol-Gemisch besteht, bei 500 mbar abgenommen. Beim Erreichen von 74,5°C, bei welcher Temperatur Methanol weitgehend entfernt ist, tritt ein Phasenzerfall des übergehenden Azeotrops, das nun weitgehend aus Methylglyoxaldimethylacetal-Wasser besteht, ein. Die Methylglyoxaldimethylacetal-Phase (8) wird unter Vollabnahme ausgekreist, und die wäßrige Unterphase (7) wird zurückgeführt, bis die Methylglyoxaldimethylacetal-Phase (8) nicht mehr zunimmt.

Aus dem Rührkessel werden unter Absenken des Vakuums auf 100 mbar anschließend 500 ml Wasserphase abdestilliert (7), die im nächsten Verfahrensschritt vor Abdestillation des Produkts in den Sumpf zurückgeführt wird.

Auch die Zwischenfraktion (5) und der Sumpf (9) werden wieder in das Verfahren zurückgeführt, wie in Beispiel 1B näher beschrieben.

Beispiel 1B

Dieses Beispiel beschreibt die Vorgehensweise einer der diskontinuierlichen Verfahrenstufen, die nicht die erste Verfahrensstufe ist. Der Sumpf (9) des vorherigen Ansatzes wird vorgelegt und mit 5,5 Mol Methylglyoxal versetzt. Dann wird, wie in Beispiel 1A, Wasser abdestilliert, und nach Abkühlen wird zurückgewonnenes Methanol 4 zugesetzt und gemäß GC-Analytik so mit frischem Methanol ergänzt, daß insgesamt 1760 g (= 55 Mol) Methanol vorliegen. Die Mischung wird dann, wie in Beispiel 1A, am Ionenaustauscher umgesetzt. Anschließend werden die Zwischenfraktionen (5) und die Wasserphase (7) des vorhergehenden Ansatzes in den Rührkessel gegeben, und es wird wie in Beispiel 1A destilliert.

Beispiel 1C

Es wurden zwei Ansätze durchgeführt, bei denen lediglich der Sumpf (9) der Destillation der letzten Stufe weiter verarbeitet und kein frisches Methylglyoxal zugesetzt wurde, wobei ansonsten wie in Beispiel 1B vorgegangen wurde.

Nach Durchführung eines wie in Beispiel 1A beschriebenen Verfahrensschritts, weiteren neun wie in Beispiel 1B beschrieben Verfahrensschritten und zwei wie in Beispiel 1C beschriebenen Verfahrensschritten, wurden insgesamt 4474,7 g 86,5%iges Roh-Methylglyoxaldimethylacetal gewonnen, was einer Gesamtausbeute an Roh-Methylglyoxaldimethylacetal von 84,4% entspricht. Die folgende Tabelle 1 gibt die Zusammensetzungen des Ansatzes und der Produkte wieder. Die MGDA 2- bzw. MGDA $\Sigma_2$-Phase (ausgeschleustes Methylglyoxaldimethylacetal) besteht zu 86,5% aus Methylglyoxaldimethylacetal, zu 9,1% aus Wasser und zu 1,6% aus Methanol (oder: ist 86,5%ig).

Tabelle 1

| Rückführung | 0. (g/%) | 1. (g/%) | 2. (g/%) | 3. (g/%) |
|---|---|---|---|---|
| MG $\Sigma_{Ein}$ | 5,5 mol | 11,0 mol | 16,5 mol | 22,0 mol |
| MGDA 1 | 296,2/45,6 | 450,6/69,4 | 482,9/74,4 | 543,7/83,8 |
| MGTA 1 | 38,0/4,2 | 35,3/3,9 | 33,7/3,7 | 28,3/3,1 |
| MGTA $\Sigma_1$ | 38,0/4,2 | 73,3/4,1 | 107,0/4,0 | 135,3/3,8 |
| MGDA $\Sigma_1$ | 296,2/45,6 | 746,8/57,5 | 1229,7/63,1 | 1773,4/68,3 |
| MGDA 2 | 92,1/14,2 | 479,6/73,9 | 514,8/79,3 | 492,6/75,9 |
| MGDA $\Sigma_2$ | 92,1/14,2 | 571,7/44,0 | 1086,5/55,8 | 1579,1/60,8 |
| Rückführung | 4. (g/%) | 5.* (g/%) | 6. (g/%) | 7. (g/%) |
| MG $\Sigma_{Ein}$ | 27,5 mol | 27,5 mol | 33,0 mol | 38,5 mol |
| MGDA 1 | 522,7/80,5 | 317,4/48,9* | 424,0/65,3 | 500,7/77,1 |
| MGTA 1 | 31,9/3,5 | 32,5/3,6* | 51,3/5,7 | 33,0/3,7 |
| MGTA $\Sigma_1$ | 167,2/3,7 | 195,2/4,3 | 246,5/4,6 | 279,5/4,4 |
| MGDA $\Sigma_1$ | 2296,1/70,8 | 2613,5/80,5 | 3037,5/78,0 | 3538,2/77,9 |
| MGDA 2 | 563,4/86,8 | 266,1/41,0* | 581,1/89,5 | 394,7/60,8 |
| MGDA $\Sigma_2$ | 2142,5/66,0 | 2408,6/74,2 | 2989,7/76,8 | 3384,4/74,5 |
| Rückführung | 8. (g/%) | 9. (g/%) | 10. (g/%) | 11.* (g/%) |
| MG $\Sigma_{Ein}$ | 44,0 mol | 49,5 mol | 55,0 mol | 55,0mol |
| MGDA 1 | 493,0/76,0 | 525,7/81,0 | 555,1/85,5 | 279,5/43,1 |
| MGTA 1 | 52,3/5,8 | 42,2/4,7 | 43,1/4,8 | 71,7/8,0* |
| MGTA $\Sigma_1$ | 331,8/4,6 | 374,0/4,6 | 417,1/4,6 | 488,8/5,4 |
| MGDA $\Sigma_1$ | 4031,2/77,6 | 4556,9/78,0 | 5112,0/78,8 | 5391,5/83,1 |
| MGDA 2 | 629,3/97,0 | 513,9/79,2 | 582,7/89,9 | 364,4/56,2 |
| MGDA $\Sigma_2$ | 4013,7/77,3 | 4527,6/77,5 | 5110,3/78,7 | 5474,7/84,4 |

MGDA 1: Ausbeute Einzelansatz nach Umpumpen über BayKat 2611 bezogen auf jeweils 5,5 mol MG
MGTA 1: Ausbeute des Einzelansatzes an 1,1,2,2-Tetramethoxypropan
MGDA $\Sigma_1$: kummulierte MGDA-Ausbeute der Einzelansätze
MGTA $\Sigma_1$: kummulierte MGTA-ausbeute der Einzelansätze
MGDA 2: ausgeschleuste MGDA-Ausbeute des Einzelausatzes
MGDA $\Sigma_2$: kummulierte ausgeschleuste MGDA-Ausbeute

*: Ansatz ohne Zusatz von MG, Ausbeute ist fiktiv auf 5,5 mol MG berechnet

Beispiel 2

Im Beispiel 2 wurden die Versuchsbedingungen variiert. In den Reaktionsansatz wurde kein hochsiedendes Lösungsmittel wie z. B. Diethylenglycol gegeben. Die Sumpftemperatur bei der Entwässerung, die wie in Beispiel 1 bie 100mbar vorgenommen wurde, wurde auf 70°C eingestellt. Der $H_2O$-Gehalt des Sumpfes lag bei etwa 10%. Nach insgesamt zwölf derartigen Verfahrensschritten betrug die Gesamtausbeute an ausgeschleustem Roh-Methylglyoxaldimethylacetal ungefähr 80%. Die folgende Tabelle 2 gibt die Daten des Beispiels 2 in Einzelheiten wieder.

**Tabelle 2**

| Verfahrensschritte Beispiel 2 | 0 | 1 | 2 | 3 | 4 | 5 | 6 |
|---|---|---|---|---|---|---|---|
| MG-Ein (kummuliert, mol) | 5,5 | 11,0 | 16,5 | 22,0 | 27,5 | 27,5 | 33,0 |
| MGDA 4 (Einzelansatz, g) | 306,0 | 399,9 | 472,3 | 464,9 | 509,9 | 288,5 | 405,8 |
| MGDA 4 (Einzelansatz, mol%) | 47,1 | 61,6 | 72,8 | 71,6 | 78,6 | 44,5 | 62,5 |
| MGTA 4 (Einzelansatz, g) | 44,6 | 47,2 | 31,2 | 38,7 | 27,6 | 19,8 | 28,8 |
| MGTA 4 (Einzelansatz, mol%) | 4,9 | 5,2 | 3,5 | 4,3 | 3,1 | 2,2 | 3,2 |
| MGTA 4 (kummuliert, g) | 44,6 | 91,8 | 123,0 | 161,7 | 189,3 | 209,1 | 237,9 |
| MGTA 4 (kummuliert, mol%) | 4,9 | 5,1 | 4,5 | 4,5 | 4,2 | 4,6 | 4,4 |
| MGDA 4 (kummuliert, g) | 306,0 | 705,9 | 1178,2 | 1643,1 | 2153,0 | 2441,5 | 2847,3 |
| MGDA 4 (kummuliert, mol%) | 47,1 | 54,4 | 60,5 | 63,3 | 66,3 | 75,2 | 73,1 |
| MGDA 7 (Einzelansatz, g) | 217,4 | 393,6 | 421,2 | 531,3 | 497,8 | 341,9 | 394,1 |
| MGDA 7 (Einzelansatz, mol%) | 33,5 | 60,6 | 64,9 | 81,9 | 76,7 | 52,7 | 60,7 |
| MGDA 7 (kummuliert, g) | 217,4 | 611,0 | 1032,2 | 1563,5 | 2061,3 | 2403,2 | 2797,3 |
| MGDA 7 (kummuliert, mol%) | 33,5 | 47,1 | 53,0 | 60,2 | 63,5 | 74,1 | 71,8 |
| | | | | | | Rückführung ohne MG-Zusatz | |

| Verfahrensschritte Beispiel 2 | 7 | 8 | 9 | 10 | 11 | |
|---|---|---|---|---|---|---|
| MG-Ein (kummuliert, mol) | 38,5 | 44,0 | 49,5 | 55,0 | 55,0 | |
| MGDA 4 (Einzelansatz, g) | 459,5 | 467,1 | 520,0 | 513,1 | 331,5 | |
| MGDA 4 (Einzelansatz, mol%) | 70,8 | 72,0 | 80,1 | 79,1 | 51,1 | |
| MGTA 4 (Einzelansatz, g) | 27,6 | 54,2 | 28,6 | 32,9 | 33,6 | |
| MGTA 4 (Einzelansatz, mol%) | 3,1 | 6,0 | 3,2 | 3,6 | 3,7 | |
| MGTA 4 (kummuliert, g) | 265,5 | 319,7 | 348,3 | 381,2 | 414,8 | |
| MGTA 4 (kummuliert, mol%) | 4,2 | 4,4 | 4,3 | 4,2 | 4,6 | |
| MGDA 4 (kummuliert, g) | 3306,8 | 3773,9 | 4293,9 | 4807,0 | 5138,5 | |
| MGDA 4 (kummuliert, mol%) | 72,8 | 72,7 | 73,5 | 74,1 | 79,2 | |
| MGDA 7 (Einzelansatz, g) | 450,8 | 465,3 | 514,0 | 521,5 | 416,4 | |
| MGDA 7 (Einzelansatz, mol%) | 69,5 | 71,7 | 79,2 | 80,4 | 64,2 | |
| MGDA 7 (kummuliert, g) | 3248,1 | 3713,4 | 4227,4 | 4748,9 | 5165,3 | |
| MGDA 7 (kummuliert, mol%) | 71,5 | 71,5 | 72,4 | 73,2 | 79,6 | |
| | | | | | **Rückführung ohne MG-Zusatz** | |

EP 0 704 422 A1

Vergleichsbeispiel

45 g wäßrige 40%ige Methylglyoxallösung (0,25 Mol Methylglyoxal), 185 g Methanol (5,78 Mol) und 12,5 g stark saurer Ionenaustauscher Dowex 50WX8 der Firma Dow-Chemical wurden 16 Stunden unter Rückfluß erhitzt. Nach Abfiltrieren vom Katalysator sind nach Analyse des Filtrats (gaschromatographisch bestimmte Gewichtsprozent, interner Standard: Dioxan) nur 16,1 g (0,14 mol, 56% bezogen auf Methylglyoxal) Methylglyoxaldimethylacetal entstanden.

**Patentansprüche**

1. Verfahren zur Herstellung von Methylglyoxaldimethylacetal aus Methylglyoxal und Methanol in Gegenwart eines Katalysators, **dadurch gekennzeichnet, daß** das gebildete Methylglyoxaldimethylacetal mit Hilfe von azeotroper destillierender Erwärmung in Gegenwart von Wasser gewonnen wird.

2. Verfahren nach Anspruch 1, wobei Methylglyoxal, das zu Beginn des Verfahrens in wäßriger Lösung mit mehr als 40 Gew.-% Wasseranteil vorliegt, vor Durchführung weiterer Verfahrensschritte durch Abdestillieren von Wasser auf eine wäßrige Methylglyoxallösung mit einem Wassergehalt von weniger als 40 Gew.-% angereichert wird.

3. Verfahren nach einem der Ansprüche 1 oder 2, dadurch gekennzeichnet, daß der Katalysator ein saurer Ionenaustauscher, bevorzugt ein Ionenaustauscherbett, ist.

4. Verfahren nach Anspruch 3, dadurch gekennzeichnet, daß das Methylglyoxal und das Methanol bei einer erhöhten Temperatur über das saure Ionenaustauscherbett gepumpt werden.

5. Verfahren nach einem der Ansprüche 2 bis 4, dadurch gekennzeichnet, daß die Methylglyoxallösung auf einen Wassergehalt von weniger als 20 Gew.-% angereichert wird.

6. Verfahren nach einem der Ansprüche 1 bis 5, dadurch gekennzeichnet, daß bei der azeotropen Gewinnung von Methylglyoxaldimethylacetal in Gegenwart von Wasser in einem Vorlauf Methanol im wesentlichen vollständig abdestilliert wird.

7. Verfahren nach einem der Ansprüche 1 bis 6, dadurch gekennzeichnet, daß das Verfahren diskontinuierlich durchgeführt wird.

8. Verfahren nach einem der Ansprüche 1 bis 6, dadurch gekennzeichnet, daß der Vorlauf und/oder der Sumpf der destillierenden Erwärmung in das Verfahren zurückgeführt werden.

9. Verfahren nach einem der Ansprüche 7 oder 8, dadurch gekennzeichnet, daß der Ionenaustauscher in das Verfahren zurückgeführt wird.

10. Verfahren nach einem der vorhergehenden Ansprüche, dadurch gekennzeichnet, daß aus der destillierenden Erwärmung ein gasförmiges Azeotrop aus Methylglyoxaldimethylacetal und Wasser kondensiert und in zwei flüssige Phasen, nämlich eine wäßrige Phase und eine Methylglyoxaldimethylacetal-Phase, getrennt wird.

11. Verfahren zur Trennung eines Gemisches aus Methylglyoxaldimethylacetal und Wasser, dadurch gekennzeichnet, daß ein azeotropes Gemisch aus Methylglyoxaldimethylacetal und Wasser destilliert wird, das destillierte und rekondensierte Gemisch in zwei flüssige Phasen, nämlich in eine wäßrige Phase und eine Methylglyoxaldimethylacetal-Phase, zerfallen lassen wird und daß die beiden Phasen voneinander getrennt werden.

Fig. 1

Fig. 2

**Europäisches Patentamt**

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,X | DE-A-29 47 383 (HOECHST) <br> * Seite 3; Beispiele * <br> --- | 1-3,5,7 | C07C49/175 <br> C07C45/64 <br> C07C45/84 |
| A | DE-B-12 52 193 (BASF) <br> * Beispiele 1,2,7-10 * <br> --- | 1-11 | |
| A | US-A-2 421 559 (H. R. GUEST ET AL) <br> * Spalte 1 - Spalte 2 * <br> ----- | 1-11 | |

**RECHERCHIERTE SACHGEBIETE (Int.Cl.6)**

C07C

Der vorliegende Recherchenbericht wurde für alle Patentansprüche erstellt

| Recherchenort | Abschlußdatum der Recherche | Prüfer |
|---|---|---|
| DEN HAAG | 18.Dezember 1995 | Wright, M |

EPO FORM 1503 03.82 (P04C03)